# DEMANDE DE BREVET EUROPEEN

(11) **EP 2 905 508 A2**
(43) Date de publication de la demande: **12.08.2015**
(21) Numéro de dépôt: 15305156.0
(22) Date de dépôt: 03.02.2015
(51) Int. Cl.: F16H 25/20

(54) **Dispositif de contrôle de la translation axiale d'un élément coulissant par rapport à une structure porteuse**

(30) Priorité: 07.02.2014 FR 1450976
(71) Demandeur: AD-HOC Scientific (Société par Actions Simplifiée à Associé Unique), 67850 Offendorf (FR)
(72) Inventeur: Sabatier, Jean-Paul, 39150 Saint Pierre (FR)
(74) Mandataire: Nuss, Laurent

(57) **Abrégé**

La présente invention a pour objet un dispositif de contrôle (1) de la translation axiale d'un élément coulissant (2) par rapport à une structure porteuse, l'élément coulissant (2) étant associé à un ensemble d'actionnement (3) de la translation disposé de façon coaxiale par rapport à l'axe de translation de l'élément coulissant (2), l'ensemble d'actionnement (3) fonctionnant par rotation d'au moins une vis sans fin (3a) selon un axe parallèle à l'axe de translation de l'élément coulissant (2), caractérisé en ce que le dispositif de contrôle (1) comprend un au moins un mécanisme de régulation (4) adapté pour entraîner en rotation l'ensemble d'actionnement (3) autour d'un axe parallèle à l'axe de translation de l'élément coulissant (2).

## Description

La présente invention se rapporte au domaine des mécanismes d'actionnement de mouvement de haute précision et plus particulièrement aux dispositifs de contrôle de l'actionnement et de la réalisation d'un mouvement de haute précision.

Actuellement, les pompes à perfusion permettent une injection contrôlée d'une solution médicamenteuse dans une poche de perfusion reliée à un patient voire même une injection directement dans le corps d'un patient. Ces pompes à perfusion se présentent sous la forme d'une seringue dont le piston est actionné par un moteur généralement piloté par un ordinateur et contrôlant le déplacement axial du piston dans le corps de la seringue pour éjecter le contenu de celle-ci.

Les pompes à perfusion étant utilisées pour des solutions médicamenteuses souvent très puissantes pouvant être dangereuses à trop forte concentration, voire même létale, il est alors impératif que la translation du piston dans la seringue soit effectuée de façon précise avec un contrôle du déplacement qui soit pratiquement nanométrique.

Pour opérer efficacement un déplacement avec une telle précision, la solution mécanique classiquement utilisée consiste à utiliser un moteur actionnant en rotation une vis sans fin dont le filetage interagit avec un taraudage complémentaire porté par l'extrémité du piston de la seringue. La rotation de la vis sans fin permet ainsi son déplacement dans le taraudage du piston et d'opérer alors le coulissement du piston dans la seringue. Le contrôle avec précision du déplacement du piston dans la seringue est alors opéré grâce à une vis et un taraudage présentant un pas de vis très réduit de sorte qu'un nombre élevé de rotation de la vis est nécessaire pour entrainer un déplacement du piston qui soit important. Dans ces conditions, le temps de travail du moteur nécessaire pour déplacer le piston est donc également très élevé, ce qui permet d'opérer des injections continues de substances médicamenteuses dans doses infinitésimales sur des périodes de plusieurs heures. De façon complémentaire, le mécanisme qui assure l'interaction de la vis avec le taraudage du piston peut parfois faire intervenir des éléments additionnels montés de façon épicycloïdale pour optimiser l'action de la vis sur la translation du piston.

Cependant, un tel mécanisme présente rapidement des limites de fonctionnement.

En effet, si un tel mécanisme permet un déplacement lent et contrôlé du piston dans la seringue lors d'une éjection de solution, un tel mécanisme nécessite également un temps important pour effectuer le remplissage de la seringue. De même, en cas d'urgence, un tel mécanisme n'est pas en mesure d'opérer un reflux rapide pour stopper une injection de médicament qui aurait été trop importante ou qui ne serait plus nécessaire.

De plus, un mécanisme faisant intervenir un pas de vis réduit impose au moteur d'être en mesure de fonctionner avec un couple important. Aussi, compte tenu des limites de fonctionnement inhérentes aux moteurs, le moteur imposera une rotation minimale et donc un déplacement minimal au piston pour pouvoir être en mesure de fonctionner.

Pour répondre à ces inconvénients, certains mécanismes ont pu être envisagés. Toutefois, ceux-ci sont généralement complexes à mettre en oeuvre et présentent l'inconvénient d'être difficilement adaptable sur des pompes à perfusion déjà existante.

La présente invention a pour but de pallier ces inconvénients en proposant un dispositif de contrôle de la translation axiale d'un élément coulissant par rapport à une structure porteuse qui permette un contrôle fin et précis du déplacement de l'élément coulissant, autorise rapidement un fonctionnement inversé et soit facile à mettre en oeuvre sur des appareillages existants.

L'invention a ainsi pour objet un dispositif de contrôle de la translation axiale d'un élément coulissant par rapport à une structure porteuse, l'élément coulissant étant associé à un ensemble d'actionnement de la translation disposé de façon coaxiale par rapport à l'axe de translation de l'élément coulissant, l'ensemble d'actionnement fonctionnant par rotation d'au moins une vis sans fin selon un axe parallèle à l'axe de translation de l'élément coulissant, caractérisé en ce que le dispositif de contrôle comprend un au moins un mécanisme de régulation adapté pour entraîner en rotation l'ensemble d'actionnement autour d'un axe parallèle à l'axe de translation de l'élément coulissant.

L'invention porte également sur différents procédés de mise en oeuvre d'un dispositif de contrôle de la translation axiale d'un élément coulissant par rapport à une structure porteuse selon l'invention.

L'invention concerne encore différentes mises en oeuvre d'un dispositif de contrôle de la translation axiale d'un élément coulissant par rapport à une structure porteuse selon l'invention.

L'invention sera mieux comprise, grâce à la description ci-après, qui se rapporte à un mode de réalisation préféré, donné à titre d'exemple non limitatif, et expliqué avec référence aux dessins schématiques annexés, dans lesquels :
- la figure 1 est une représentation schématique d'un exemple de dispositif de contrôle selon l'invention appliqué à un mécanisme de translation d'un élément coulissant selon une vue éclatée,

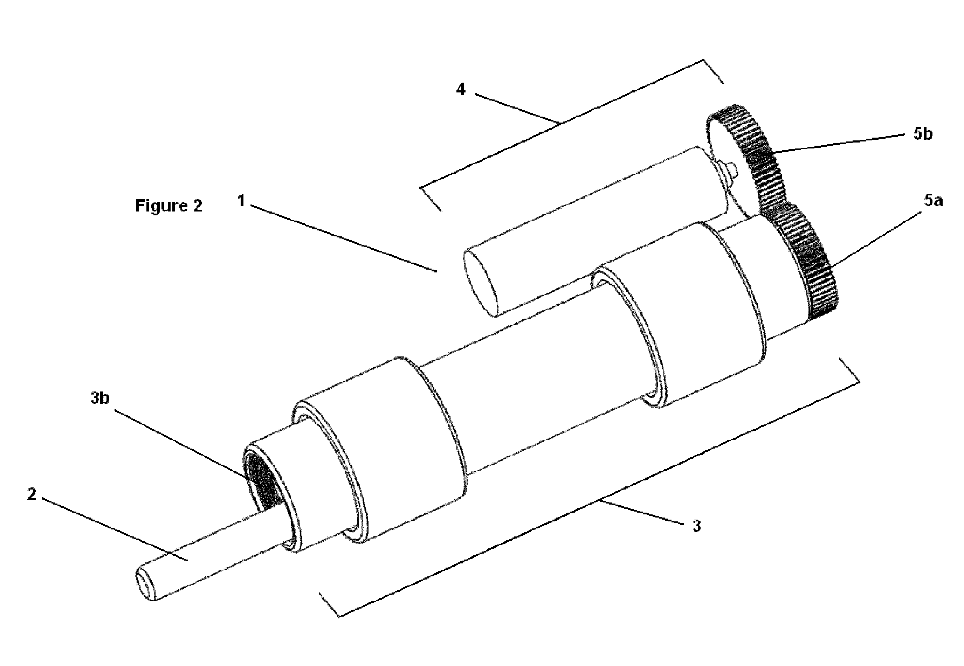

- la figure 2 est une représentation schématique d'un exemple de dispositif de contrôle selon l'invention appliqué à un mécanisme de translation d'un élément coulissant selon une vue extérieure.

La présente invention porte sur un dispositif de contrôle 1 de la translation axiale d'un élément coulissant 2 par rapport à une structure porteuse, l'élément coulissant 2 étant associé à un ensemble d'actionnement 3 de la translation disposée de façon coaxiale par rapport à l'axe de translation de l'élément coulissant 2, l'ensemble d'actionnement 3 fonctionnant par rotation d'au moins une vis sans fin 3a selon un axe parallèle à l'axe de translation de l'élément coulissant 2, caractérisé en ce que le dispositif de contrôle 1 comprend un au moins un mécanisme de régulation 4 adapté pour entraîner en rotation l'ensemble d'actionnement 3 autour d'un axe parallèle à l'axe de translation de l'élément coulissant 2.

Le contrôle de la translation axiale par le dispositif 1 de l'invention porte notamment sur le contrôle de la vitesse et de l'accélération du déplacement de cet élément coulissant 2. Le contrôle de la translation se rapporte également à un contrôle du couple et/ou de la force générée par cet élément coulissant 2 ou au niveau d'une pièce d'actionnement de cet élément coulissant 2.

Il convient de noter que l'ensemble d'actionnement 3 de la translation de l'élément coulissant 2 comprenant au moins une vis sans fin 3a peut être réalisée de différentes façons sans que cela n'affecte le dispositif de contrôle 1 de l'invention. En effet, le dispositif de contrôle 1 de l'invention est adaptable à tout type d'actionnement 3 de la translation d'un élément coulissant 2, dès l'instant que cet actionnement fait intervenir une vis sans fin 3a interagissant, directement ou indirectement, avec un filet taraudé 3b et que cette vis sans fin 3a présente un positionnement selon un axe parallèle, voire coaxiale, à l'axe de translation de l'élément coulissant 2.

Le principe de fonctionnement du dispositif de contrôle 1 de l'invention consiste opérer une rotation de l'ensemble d'actionnement 3 de façon à influer la rotation de la vis sans fin 3a de celui par rapport à l'élément coulissant 2. Par ce principe, le dispositif de contrôle 1 permet d'agir sur l'effet de l'ensemble d'actionnement 3 en fonctionnement en amplifiant, en atténuant, en annulant, voire en inversant l'action de la vis sans fin 3a de l'ensemble d'actionnement 3 sur un filet taraudé permettant le déplacement de l'élément coulissant 2.

Selon une particularité de construction, le dispositif de contrôle 1 de la translation axiale d'un élément coulissant 2 selon l'invention est caractérisé en ce que, le mécanisme de régulation 4 comprend un engrenage 5 faisant coopérer des structures dentées 5a et 5b respectivement associées, d'une part, au mécanisme de régulation 4 et, d'autre part, à l'ensemble d'actionnement 3.

Selon une spécificité de construction de cette particularité, l'engrenage 5 est réalisé de sorte que les structures dentées 5a et 5b forment respectivement tout ou partie de pignons de façon à interagir directement entre eux.

Selon une spécificité alternative de construction de cette particularité, l'engrenage 5 est réalisé de sorte que les structures dentées 5a et 5b sont conduites à fonctionner par l'intermédiaire d'une liaison d'entraînement de type chaîne ou courroie. De même, cette liaison d'entraînement peut être formée par un mécanisme d'engrenage faisant intervenir un ou plusieurs pignons intermédiaires entre les deux structures dentées 5a et 5b portées par le mécanisme de régulation 4 et l'ensemble d'actionnement 3.

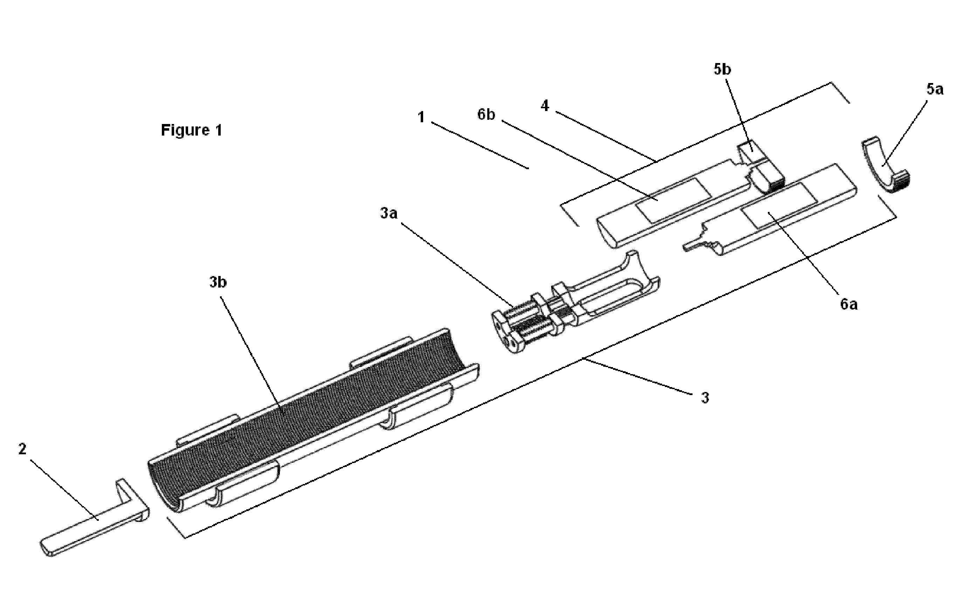

Selon un mode de construction préféré de cette particularité de réalisation, les structures dentées 5a et 5b coopérantes sont réalisées par des pignons montés-associés ou structurellement intégrés à une surface périphérique du mécanisme de régulation 4 ou de l'ensemble d'actionnement 3. Cette construction par intégration de la structure dentée 5a et/ou 5b d'au moins un des éléments de l'engrenage 5 dans l'architecture de l'élément auquel la structure 5a et/ou 5b est associée permet de limiter le nombre de pièces nécessaires pour la construction du dispositif. De même, cette intégration permet de supprimer d'éventuelles pertes de force par frottement qui peuvent survenir entre deux pièces montées serrées entre elles. Un exemple de réalisation représenté sous la forme d'une vue éclatée se trouve illustrée par la figure 1.

Il convient de noter, bien que ce ne soit pas limitatif de l'invention, que le rapport d'engrenage préférentiellement utilisé entre les deux structures dentées 5a et 5b est de 1 afin de faciliter la mise en oeuvre et le fonctionnement du dispositif de l'invention. Dans une telle configuration, les pignons 5a et 5b présentent des diamètres identiques.

Selon un mode de construction préféré de l'invention, le dispositif de contrôle 1 de la translation axiale d'un élément coulissant 2 est caractérisé en ce que, le mécanisme de régulation 4 et l'ensemble d'actionnement 3 comportant des moteurs 6a et 6b respectifs, le dispositif de contrôle 1 comprend une interface de couplage du fonctionnement du moteur 6a du mécanisme de régulation 4 par rapport à l'actionnement du moteur 6b de l'ensemble d'actionnement 3 en fonction de la longueur et/ou de la vitesse et/ou de l'accélération de la translation recherchées de l'élément coulissant 2 et/ou du couple de rotation d'au moins une vis sans fin 3a de l'ensemble d'actionnement 3 en interaction avec l'élément coulissant 2 et/ou de la force de translation recherchée de l'élément coulissant 2.

Ce couplage des moteurs 6a et 6b respectifs du mécanisme de régulation 4 et de l'ensemble d'actionnement 3 permet de contrôler la coopération des moteurs 6a et 6b qui agissent sur l'importance de la rotation de la vis sans fin 3a par rapport au filet taraudé 3b avec lequel elle interagit, directement ou indirectement. Ainsi, le moteur 6a du mécanisme de régulation 4 entraîne en rotation un élément de l'ensemble d'actionnement 3, par exemple le filet taraudé 3b ou encore le moteur 6b de celui-ci sur lequel est montée la vis sans fin 3a. En entraînant en rotation un des éléments, vis 3a ou filet taraudé 3b, de l'ensemble d'actionnement 3 qui assure le déplacement de l'élément coulissant 2, le moteur 6a du mécanisme de régulation 4, selon son sens et sa vitesse par rapport au moteur 6b de l'ensemble d'actionnement 3, est en mesure d'amplifier, d'atténuer, d'annuler, voire même d'inverser le déplacement relatif de la vis sans fin 3a par rapport au filet taraudé 3b.

Selon une particularité de construction non-limitative de l'invention, le dispositif de contrôle 1 de la translation axiale d'un élément coulissant 2 est caractérisé en ce que, d'une part, l'ensemble d'actionnement 3 interagit avec une première structure fixe en translation avec la structure porteuse, cette première structure étant susceptible d'être mise en rotation coaxiale avec l'ensemble d'actionnement 3 sous l'action du mécanisme de régulation 4 et en ce que, d'autre part, le mécanisme de régulation 4 interagit avec une seconde structure fixe en translation avec la structure porteuse.

Selon une particularité de construction non-limitative de l'invention, lorsque la pièce qui porte le filet taraudé 3b est amenée à être en rotation sous l'action du moteur 6a du mécanisme de régulation 4, cette rotation par rapport à la structure porteuse peut être gérée par l'intermédiaire d'un ou de plusieurs roulements, par exemple à billes ou à aiguilles.

L'invention porte également sur un procédé de mise en oeuvre d'un dispositif de contrôle 1 de la translation axiale d'un élément coulissant 2 par rapport à une structure porteuse selon l'invention, caractérisé en ce que ce procédé comprend au moins une étape de mise en rotation d'un élément de l'ensemble d'actionnement 3 par le mécanisme de régulation 4 réalisée de façon à amplifier le déplacement relatif de la vis sans fin 3a par rapport au filet taraudé 3b et donc augmenter la vitesse de translation axiale de l'élément coulissant 2.

L'invention concerne également un procédé de mise en oeuvre d'un dispositif de contrôle 1 de la translation axiale d'un élément coulissant par rapport à une structure porteuse selon l'invention, caractérisé en ce qu'il comprend au moins une étape de mise en rotation d'un élément de l'ensemble d'actionnement 3 par le mécanisme de régulation 4 réalisée de façon à atténuer le déplacement relatif de la vis sans fin 3a par rapport au filet taraudé 3b et donc réduire la vitesse de translation axiale de l'élément coulissant 2.

Selon une particularité de ce procédé de mise en oeuvre, la rotation d'un élément de l'ensemble d'actionnement 3 par le mécanisme de régulation est réalisée de façon à annuler le déplacement relatif de la vis sans fin 3a par rapport au filet taraudé 3b et donc stopper la translation axiale de l'élément coulissant 2.

Selon un premier mode d'application particulier de l'invention, la mise en oeuvre d'un dispositif de contrôle 1 de la translation axiale d'un élément coulissant 2 par rapport à une structure porteuse selon l'invention est opérée dans un appareil de fraisage.

Selon un second mode d'application particulier de l'invention, la mise en oeuvre d'un dispositif de contrôle 1 de la translation axiale d'un élément coulissant 2 par rapport à une structure porteuse selon l'invention est opérée dans une pompe à perfusion.

D'autres modes d'application particuliers de l'invention peuvent être envisagés.

Bien entendu, l'invention n'est pas limitée au mode de réalisation décrit et représenté aux dessins annexés. Des modifications restent possibles, notamment du point de vue de la constitution des divers éléments ou par substitution d'équivalents techniques, sans sortir pour autant du domaine de protection de l'invention.

## Revendications

1. Dispositif de contrôle (1) de la translation axiale d'un élément coulissant (2) par rapport à une structure porteuse, l'élément coulissant (2) étant associé à un ensemble d'actionnement (3) de la translation disposé de façon coaxiale par rapport à l'axe de translation de l'élément coulissant (2), l'ensemble d'actionnement (3) fonctionnant par rotation d'au moins une vis sans fin (3a) selon un axe parallèle à l'axe de translation de l'élément coulissant (2), **caractérisé en ce que** le dispositif de contrôle (1) comprend un au moins un mécanisme de régulation (4) adapté pour entraîner en rotation l'ensemble d'actionnement (3) autour d'un axe parallèle à l'axe de translation de l'élément coulissant (2).

2. Dispositif de contrôle (1) de la translation axiale d'un élément coulissant (2) par rapport à une structure porteuse selon la revendication 1, **caractérisé en ce que** le mécanisme de régulation (4) comprend un engrenage (5) faisant coopérer des structures dentées (5a et 5b) respectivement associées, d'une part, au mécanisme de régulation (4) et, d'autre part, à l'ensemble d'actionnement (3).

3. Dispositif de contrôle (1) de la translation axiale d'un élément coulissant (2) par rapport à une structure porteuse selon une des revendications précédentes, **caractérisé en ce que**, le mécanisme de régulation (4) et l'ensemble d'actionnement (3) comportant des moteurs (6a et 6b) respectifs, le dispositif de contrôle (1) comprend une interface de couplage du fonctionnement du moteur (6a) du mécanisme de régulation (4) par rapport à l'actionnement du moteur (6b) de l'ensemble d'actionnement (3) en fonction de la longueur et/ou de la vitesse et/ou de l'accélération de la translation recherchées de l'élément coulissant (2) et/ou du couple de rotation d'au moins une vis sans fin (3a) de l'ensemble d'actionnement (3) en interaction avec l'élément coulissant (2) et/ou de la force de translation recherchée de l'élément coulissant (2).

4. Dispositif de contrôle (1) de la translation axiale d'un élément coulissant (2) par rapport à une structure porteuse selon une des revendications précédentes, **caractérisé en ce que**, d'une part, l'ensemble d'actionnement (3) interagit avec une première structure fixe en translation avec la structure porteuse, cette première structure étant susceptible d'être mise en rotation coaxiale avec l'ensemble d'actionnement (3) sous l'action du mécanisme de régulation (4) et **en ce que**, d'autre part, le mécanisme de régulation (4) interagit avec une seconde structure fixe en translation avec la structure porteuse.

5. Dispositif de contrôle (1) de la translation axiale d'un élément coulissant (2) par rapport à une structure porteuse selon au moins la revendication 2, **caractérisé en ce que** les structures dentées (5a et 5b) coopérantes sont réalisées par des pignons (5a et 5b) montés-associés ou structurellement intégrés à une surface périphérique du mécanisme de régulation (4) ou de l'ensemble d'actionnement (3).

6. Dispositif de contrôle (1) de la translation axiale d'un élément coulissant (2) par rapport à une structure porteuse selon au moins la revendication 5, **caractérisé en ce que** les pignons (5a et 5b) présentent des diamètres identiques.

7. Procédé de mise en oeuvre d'un dispositif de contrôle (1) de la translation axiale d'un élément coulissant (2) par rapport à une structure porteuse selon une des revendications 1 à 6, **caractérisé en ce que** le procédé comprend au moins une étape de mise en rotation d'un élément de l'ensemble d'actionnement (3) par le mécanisme de régulation 4 réalisée de façon à amplifier le déplacement relatif de la vis sans fin 3a par rapport au filet taraudé (3b) et donc augmenter la vitesse de translation axiale de l'élément coulissant (2).

8. Procédé de mise en oeuvre d'un dispositif de contrôle (1) de la translation axiale d'un élément coulissant (2) par rapport à une structure porteuse selon une des revendications 1 à 6, **caractérisé en ce que** le procédé comprend au moins une étape de mise en rotation d'un élément de l'ensemble d'actionnement (3) par le mécanisme de régulation (4) réalisée de façon à atténuer le déplacement relatif de la vis sans fin (3a) par rapport au filet taraudé (3b) et donc réduire la vitesse de translation axiale de l'élément coulissant (2).

9. Procédé de mise en oeuvre d'un dispositif de contrôle (1) de la translation axiale d'un élément coulissant (2) par rapport à une structure porteuse selon la revendication 8, **caractérisé en ce que** la rotation d'un élément de l'ensemble d'actionnement (3) par le mécanisme de régulation est réalisée de façon à annuler le déplacement relatif de la vis sans fin (3a) par rapport au filet taraudé (3b) et donc stopper la translation axiale de l'élément coulissant (2).

10. Mise en oeuvre d'un dispositif de contrôle 1 de la translation axiale d'un élément coulissant 2 par rapport à une structure porteuse selon une des revendications 1 à 6 dans un appareil de fraisage.

11. Mise en oeuvre d'un dispositif de contrôle 1 de la translation axiale d'un élément coulissant 2 par rapport à une structure porteuse selon une des revendications 1 à 6 dans une pompe à perfusion.
